# EUROPÄISCHE PATENTSCHRIFT

(11) **EP 3 082 963 B1**
(45) Veröffentlichungstag und Bekanntmachung des Hinweises auf die Patenterteilung: **25.07.2018**
(21) Anmeldenummer: 14802393.0
(22) Anmeldetag: 18.11.2014
(51) Int. Cl.: A61Q 5/06, A61K 8/81, A61K 8/04

(54) **STYLINGSPRAY MIT VOLUMENEFFEKT**
STYLINGSPRAY WITH VOLUME EFFECT
SPRAY POUR LA MISE EN FORME DES CHEVEUX A EFFET VOLUME

(30) Priorität: 16.12.2013 DE 102013226048
(43) Veröffentlichungstag der Anmeldung: 26.10.2016
(73) Patentinhaber: Henkel AG & Co. KGaA, 40589 Düsseldorf (DE)
(72) Erfinder: KNAPPE, Thorsten, 22869 Schenefeld (DE); KAFTAN, Pamela, 22525 Hamburg (DE)
(86) Internationale Anmeldenummer: PCT/EP2014/074917
(87) Internationale Veröffentlichungsnummer: WO 2015/090803

(56) Entgegenhaltungen:
- EP-A1- 1 504 744
- DATABASE GNPD [Online] MINTEL; Juli 2013 (2013-07), "Reassuringly Firm Session Hold Hairspray", XP002734629, Database accession no. 2129335
- DATABASE GNPD [Online] MINTEL; September 2010 (2010-09), "Airy Wave Silhouette Spray Wax", XP002734630, Database accession no. 1387448
- DATABASE GNPD [Online] MINTEL; September 2011 (2011-09), "Spray Wax", XP002734631, Database accession no. 1629424
- DATABASE GNPD [Online] MINTEL; September 2011 (2011-09), "Texture.Master Texture Mist", XP002734632, Database accession no. 1633576
- DATABASE GNPD [Online] MINTEL; April 2011 (2011-04), "Ultra Shaping Hairspray Plus", XP002734633, Database accession no. 1526720
- DATABASE GNPD [Online] MINTEL; Mai 2009 (2009-05), "Shaper Plus Hairspray", XP002734634, Database accession no. 1102530

## Beschreibung

Die Anmeldung betrifft das technische Fachgebiet der temporären Umformung keratinhaltiger Fasern, insbesondere menschlicher Haare. Gegenstand der Anmeldung sind Aerosolzusammensetzungen, enthaltend ein kosmetisches Stylingmittel mit guten Eigenschaften und mindestens ein Treibmittel, wie in den Ansprüchen beschrieben, sowie die Verwendung dieser Zusammensetzungen zur temporären Umformung keratinhaltiger Fasern und ein entsprechendes Anwendungsverfahren.
Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.
Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Während bei der permanenten Umformung die chemische Struktur der keratinhaltigen Faser durch Reduktion und Oxidation modifiziert wird, findet bei der temporären Umformung keine solche Modifikation der chemischen Struktur statt. Die temporäre Formgebung soll einen guten Halt ergeben, ohne das gesunde Aussehen der Haare, wie zum Beispiel deren Glanz, zu beeinträchtigen. Entsprechende Mittel zur temporären Verformung keratinischer Fasern, im Folgenden auch Stylingmittel genannt, enthalten als festigenden Wirkstoff üblicherweise synthetische Polymere und/oder Wachse.
Stylingmittel können beispielsweise als Haarspray, Haarwachs, Haargel, Haarschaum konfektioniert werden. Insbesondere die Applikation mittels Aerosolabgabebehälter in Form eines Sprays oder eines Schaum erfreut sich hoher Beliebtheit, weil die Applikation aus Sprüh- oder Pumpbehältern ist leicht und sauber möglich.
Neben einem hohen Haltegrad müssen Stylingmittel jedoch eine ganze Reihe weiterer Anforderungen erfüllen. Zu nennen sind insbesondere die Glanzverleihung, die Volumenleistung und die Verbesserung des Griffs der Haare.
In EP1307175B1 ist ein Haarstylingmittel, umfassend ein nicht-starres emulsionspolymerisiertes quervernetztes Silikonpolymer und ein weiteres Copolymer offenbart. Dieses Stylingmittel ist in der Form einer Aerosol-Haar-Styling-Mousse oder einer Haar-Styling-Creme oder Gel fraktioniert und bietet exzellente Stil-Schöpfung (hohen Haltegrad) sowie sensorisches Gefühl (guten Griff der Haare).

In US8574553B2 ist eine haarkosmetische Zusammensetzung mit einem Wassergehalt von weniger als 10 Gew.-% offenbart, welche in einer Aerosolvorrichtung verpackt ist. Die Zusammensetzung umfasst mindestens ein anionisches fixierendes Polymer, mindestens ein Polyol, mindestens einen flüssigen Fettalkohol, mindestens einen C1-C4 Monoalkohol und ein oder mehrere Treibmittel. Gemäß US8574553B2 kann diese Zusammensetzung die Frisur gut halten und gleichzeitig das Volumen und den Glanz der Haare verbessern.

Weitere Haarstylingmittel in Aerosolform enthaltend Polyole und Carbonsäure-haltige Haftpolymere sind bekannt z.B. aus der EP 1504744 A1 oder das "Shaper Plus Hairspray" der Firma Sebastian (Mintel GNPD Datenbank, Zugangsnummer 1102530). Obgleich spezielle Stylingmittel jeweils mindestens einem der obigen Anforderungen Genüge getan haben, hat jedoch noch kein einziges bislang alle diese Anforderungen in einem optimalen Grad gezeigt.
Außerdem ist normalerweise die Zugabe mehrerer Polymere erforderlich, um die verschiedenen Anforderungen zu erzielen. Werden allerdings zu viele verschiedene Polymere eingesetzt, kann das eine Reihe von Nachteilen mit sich bringen. So können Probleme bei der Formulierung entstehen, etwa weil die Polymere untereinander oder mit anderen Bestandteilen des Mittels reagieren und es zu Ausfällungen oder Zersetzungen kommt. Bestimmte Polymere neigen auch dazu, sich auf der Haut und insbesondere auf dem Haar so beständig abzuscheiden, dass sie bei einer gewöhnlichen Wäsche nicht mehr vollständig entfernt werden und es zu einer unerwünschten Anreicherung des Polymers und damit letztlich einer Belastung von Haut oder Haar kommt.
Stylingmittel zu entwickeln, die alle gewünschten Eigenschaften/Anforderungen, nämlich den hohen Haltegrad, die Glanzverleihung, die Volumenleistung und die Verbesserung des Griffs der Haare in Kombination aufweisen, ohne dass dabei auf die Kompatibilität und die leichte Entfernbarkeit verzichtet werden müsste, bereitet nach wie vor Schwierigkeiten, besonders wenn die Applikation in einer leichten und sauberen Form wie z.B. Aerosolform fraktioniert werden soll. Der Grund dafür ist, dass das Aerosolprodukt sich auf der keratinhaltigen Faser gut verteilen lassen muss, d.h. bei Produkten in Form eines Aerosolsprays muss sich die Zusammensetzung zielgerichtet als feiner Sprühnebel gleichmäßig aufbringen lassen.
Somit sollten die in solchem Stylingmittel verwendeten Polymere in nicht wässrigen bzw. wasserfreien organischen Lösungsmittel löslich sein, doch sollten die aus solchen Stylingmittel erhaltenen Filme üblicherweise entweder wasserlöslich oder in Wasser dispergierbar sein, um ihre leichte Entfernung von dem Haar zu erleichtern. Außerdem sollten solche Polymere mit den Lösungsmitteln und/oder Treibmitteln, die üblicherweise in diesem Stylingmittel verwendet werden, vollständig verträglich sein. Weiterhin sollten die verwendeten Polymere wenig oder keine Neigung haben, sich mit den Aromastoffen oder anderen gegebenenfalls vorliegenden Bestandteilen in Stylingmittel umsetzen.
Aufgabe der vorliegenden Erfindung war es daher, ein lagerstabiles Mittel zur temporären Verformung keratinhaltiger Fasern zur Verfügung zu stellen, das sich durch einen hohen Haltegrad auszeichnet und gleichzeitig den Glanz der Frisur, das Volumen und den Griff der Haare verbessert.

Das Mittel soll sich insbesondere als feiner, zielgerichteter Sprühnebel auf die Faser applizieren lassen, und durch Wasser und/oder Seife oder Shampoo leicht entfernbar sein sollten. Bei Lagerung in einem metallischen Aerosolbehälter sollte nur eine geringe, am besten keine Korrosionsneigung durch die Konfektionierung des Mittels verursacht werden.
Ein erster Gegenstand der vorliegenden Anmeldung ist daher eine Aerosolzusammensetzung, umfassend
a) ein kosmetisches Stylingmittel, enthaltend
   a1) Glycerin,
   a2) Carbonsäuregruppen-haltiges Polymer, wobei es sich bei dem Carbonsäuregruppen-haltigen Polymer zu 100 Gew.-% um ein Copolymer gebildet aus den Monomeren
      a2i) N-tert-Octylacrylamid
      a2ii) Acrylsäure
      a2iii) tert.-Butylaminoethylmethacrylsäure
         sowie gegebenenfalls weiteren Monomeren handelt,
   dadurch gekennzeichnet, dass das Verhältnis der Gewichtsanteile von Komponente a1) und Komponente a2) in dem Mittel größer als 1 beträgt,
b) ein Treibmittel oder Treibmittelgemisch.
Ein wesentlicher Bestandteil der erfindungsgemäßen Aerosolzusammensetzungen ist das in dem kosmetischen Stylingmittel enthaltene Polymer, welches mindestens eine Carbonsäuregruppe aufweist und im Sinne der Erfindung im Rahmen der temporären Umformung keratinhaltiger Fasern zum Halt der aufgeprägten Form der Fasern (bei Haaren insbesondere der Halt einer Frisur oder des Haarvolumens) beiträgt. Als eine Testmethode für die festigende Wirkung eines Wirkstoffs wird häufig der so genannte curl-retention - Test angewendet.
Bevorzugte Polymer a2) basieren auf mindestens einem Monomer aus der Gruppe Acrylsäure, Methacrylsäure, C₁-C₆-Alkylacrylsäureester, C₁-C₆-Alkylmethacrylsäureester. Bei den Acrylsäureestern und Methacrylsäureestern handelt es sich vorzugsweise um Ester der jeweiligen Säuren mit nicht-tertiären Alkylalkoholen mit Alkylresten von 1 bis 12 Kohlenstoffatomen, insbesondere 2 bis 4 Kohlenstoffatomen. Als geeignete Monomere seien beispielsweise Ethylacrylat, Ethylmethacrylat, Propylacrylat, Propylmethacrylat, n-Butylacrylat, n-Butylmethacrylat, Isobutylacrylat, 2-Methylbutylacrylat, 2-Ethylhexylacrylat, n-Octylacrylat, Isooctylacrylat, Isooctylmethacrylat, Isononylacrylat und Isodecylacrylat genannt.
Zur Gruppe dieser filmbildenden und/oder festigenden Polymere a2) zählt u.a. ein Copolymer gebildet aus den Monomeren N-tert-Octylacrylamid, Acrylsäure, tert.-Butylaminoethylmethacrylsäure sowie gegebenenfalls weiteren Monomeren, welches zu mindestens 50 Gew.-% im Polymere a2) vorhanden sein muss. Das zuvor beschriebene Copolymer wird beispielsweise unter der Bezeichnung Amphomer® (INCI-Bezeichnung: Octylacrylamide / Acrylates / Butylaminoethyl Methacrylate Copolymer) von der Firma National Starch vertrieben.

Eine weitere ganz besonders bevorzugte erfindungsgemäße Aerosolzusammensetzung enthält als Komponente a2) mindestens ein unter der INCI-Bezeichnung: Octylacrylamide / Acrylates / Butylaminoethyl Methacrylate Copolymer bekanntes Polymer. Eine weitere ganz besonders bevorzugte erfindungsgemäße Aerosolzusammensetzung enthält als Komponente a2) ausschließlich ein unter der INCI-Bezeichnung: Octylacrylamide / Acrylates / Butylaminoethyl Methacrylate Copolymer bekanntes Polymer. Der Einsatz dieses Polymers hat sich als für die kosmetischen Eigenschaften und die Lagerstabilität der erfindungsgemäßen Mittel vorteilhaft erwiesen.
Im Sinne der Erfindung berechnen sich Gewichtsmengenangaben, die sich auf das "Stylingmittel" beziehen, auf das Gesamtgewicht des Stylingmittels (d.h. ohne das Treibmittel/Treibmittelgemisch). Eine erfindungsgemäß bevorzugte Aerosolzusammensetzung ist dadurch gekennzeichnet, dass der Gewichtsanteil der Komponente a2) am Gesamtgewicht des kosmetischen Stylingmittels zwischen 0,1 und 20 Gew.-%, vorzugsweise zwischen 0,5 und 15,0 Gew.-% und insbesondere zwischen 1,0 und 10,0 Gew.-% beträgt.
Ein weiterer wesentlicher Bestandteil der erfindungsgemäßen Aerosolzusammensetzungen ist der in dem kosmetischen Stylingmittel enthaltene Polyalkohol, ausgewählt aus der Gruppe, bestehend aus Glycerin. Solche Polyalkohole a1) sind Weichmacher der Polymere a2), und erhöhen die Flexibilität des bei Anwendung der erfindungsgemäßen Zusammensetzung gebildeten Polymerfilms. Die erfindungsgemäße Aerosolzusammensetzung enthält in dem kosmetischen Stylingmittel als Komponente a1) Glycerin.
Die erfindungsgemäße Aerosolzusammensetzung ist dadurch gekennzeichnet, dass der Gewichtsanteil der Komponente a1) am Gesamtgewicht des kosmetischen Stylingmittels zwischen 0,5 und 30 Gew.-%, vorzugsweise zwischen 1,0 und 25 Gew.-% und insbesondere zwischen 2,0 und 15 Gew.-% beträgt.

Als für die kosmetischen Eigenschaften der erfindungsgemäßen Aerosolzusammensetzung besonders vorteilhaft hat sich ein Verhältnis der Gewichtsanteile von Komponente a1) und Komponente a2) in dem kosmetischen Stylingmittel zwischen 1:1 und 20:1, vorzugsweise zwischen 2:1 und 10:1 erwiesen. Aerosolzusammensetzungen, in denen das Verhältnis der Gewichtsanteile von Komponente a1) und Komponente a2) in dem kosmetischen Stylingmittel zwischen 1:1 und 20:1, vorzugsweise zwischen 2:1 und 10:1 beträgt, werden daher erfindungsgemäß bevorzugt.
Eine weitere ganz besonders bevorzugte erfindungsgemäße Aerosolzusammensetzung enthält in dem kosmetischen Stylingmittel als Komponente a1) Glycerin und als Komponente a2) Octylacrylamide / Acrylates / Butylaminoethyl Methacrylate Copolymer, wobei das Verhältnis der Gewichtsanteile von Komponente a1) und Komponente a2) in dem Mittel zwischen 2:1 und 10:1 beträgt.
Es sollte beachtet werden, dass die erfindungsgemäß verwendeten Polymere teilweise neutralisiert werden können, bevor sie in die fertigen Stylingmittel eingearbeitet werden, wodurch es gestattet wird, dass sie aus dem Haar durch ein einfaches Spülen mit Wasser entfernt werden. Dies kann in der Weise geschehen, dass man die Polymere in Form einer Lösung in einem organischen Lösungsmittel mit oder ohne Zusatz von Wasser mit einem alkalischen Reagenz einer Konzentration umsetzt, welche auf molarer Grundlage einem Minimum von etwa 50% der verfügbaren Carboxylgruppen, die in den Polymeren vorhanden sind, äquivalent ist. Geeignete alkalische Materialien, die hierzu verwendet werden können, sind z.B. Natrium- und Kaliumhydroxid, Ammoniak, primäre, sekundäre und tertiäre Amine, Alkanolamine und Hydroxyamine wie Aminomethyl Propanol und Aminomethyl Propandiol. Werden die Polymere nicht auf diese Weise vorneutralisiert werden, dann kann ihre eventuelle Entfernung immer noch in einfacher Weise vorgenommen werden, indem man eine schwach alkalische wässrige Lösung, z.B. eine Seifenlösung verwendet.
Eine erfindungsgemäß bevorzugte Aerosolzusammensetzung enthält in dem kosmetischen Stylingmittel, bezogen auf sein Gesamtgewicht, insgesamt 0,05 bis 10 Gew.-%, vorzugsweise 0,1 bis 8 Gew.-%, weiter bevorzugt 0,2 bis 5 Gew.-% und insbesondere 0,3 bis 2 Gew.-% alkalische Materialien, vorzugsweise Aminomethyl Propanol.
Als weiteren Bestandteil neben dem kosmetischen Stylingmittel a) umfasst die erfindungsgemäße Aerosolzusammensetzung ein Treibmittel(gemisch) b).
Die erfindungsgemäß bevorzugten Treibmittel sind ausgewählt aus den Kohlenwasserstoffen mit 3 bis 5 Kohlenstoffatomen, wie Propan, n-Butan, iso-Butan, n-Pentan und iso-Pentan, Dimethylether, Kohlendioxid, Distickstoffoxid, Fluorkohlenwasserstoffen und Fluorchlorkohlenwasserstoffen sowie Mischungen dieser Substanzen. Ganz besonders bevorzugte Treibmittel sind Propan, Butan, Isobutan, Pentan, Isopentan, Dimethylether und die Gemische dieser zuvor genannten Treibmittel jeweils untereinander.

Gemäß einer bevorzugten Ausführungsform enthält die erfindungsgemäße Aerosolzusammensetzung die genannten Kohlenwasserstoffe, Dimethylether oder Mischungen der genannten Kohlenwasserstoffe mit Dimethylether als einziges Treibmittel. Die Erfindung umfasst aber ausdrücklich auch die Mitverwendung von Treibmittel vom Typ der Fluorchlorkohlenwasserstoffe, insbesondere aber der Fluorkohlenwasserstoffe.

Eine erfindungsgemäß bevorzugte Aerosolzusammensetzung ist dadurch gekennzeichnet, dass das Treibmittel oder Treibmittelgemisch aus Dimethylether, Alkanen mit 3 bis 5 Kohlenstoffatomen und Fluorkohlenwasserstoffe ausgewählt wird.

Eine besonders bevorzugte Aerosolzusammensetzung ist dadurch gekennzeichnet, dass das Treibmittel oder Treibmittelgemisch in Mengen von 10 bis 85 Gew.-%, vorzugsweise 20 bis 80 Gew.-% und insbesondere 35 bis 60 Gew.-%, bezogen auf die gesamte Aerosol-zusammensetzung eingesetzt wird.

Die erfindungsgemäße Aerosolzusammensetzung wird vorzugsweise in einem Druckbehälter konfektioniert. Ein "Druckbehälter" ist erfindungsgemäß ein Behälter, der im Inneren einen höheren Gasdruck aufweist als außerhalb des Behälters und aus dem sich ein Gasstrom über ein Ventil entnehmen lässt. Druckbehälter, mit deren Hilfe ein Produkt (z.B. eine flüssige Zusammensetzung) durch den inneren Gasdruck des Behälters über ein Ventil abgegeben wird, bezeichnet man definitionsgemäß als "Aerosolabgabebehälter" oder "Aerosolbehälter".

Die erfindungsgemäßen Aerosolzusammensetzungen lassen sich in üblicher Art und Weise herstellen. In der Regel werden alle Bestandteile der Zubereitung der erfindungsgemäßen Aerosolzusammensetzung in einen geeigneten druckfesten Behälter eingefüllt. Dieser wird daraufhin mit einem geeigneten Sprühkopf/Ventil verschlossen. Über herkömmliche Techniken wird schließlich die gewünschte Menge des speziellen Treibmittels eingefüllt.

Als druckfeste Behälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber dem im Druckbehälter konfektionisierten Mittel. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein.

Die erfindungsgemäßen Zusammensetzungen können auch in einem Mehrkammerspender verpackt sein. Der Mehrkammerspender kann auch so eingesetzt werden, dass eine Kammer das komprimierte Treibmittel und eine andere Kammer mit den restlichen Bestandteilen der erfindungsgemäßen Aerosolzusammensetzung befüllt ist. Ein derartiger Mehrkammerspender ist beispielsweise eine so genannte Bag-in-Can-Verpackung.

Die Aerosolbehälter weisen bevorzugt ein Stem-Ventil mit einer Stembohrung von 1 x 0,2 mm bis 1 x 0,7 mm auf.

Die Ventile weisen wiederum bevorzugt eine VPH-Bohrung von 0,00 mm bis 0,60 mm auf.

Die Ventile weisen wiederum bevorzugt einen RTP Durchmesser von 0,30 bis 1,60 mm auf.

Hierbei sind folgende Ventile erfindungsgemäß bevorzugt geeignet:
- Coster Typ KPM (Stembohrung von 1 x 0,27 mm bis 1 x 0,60 mm)
- Coster Typ KRA (Stembohrung von 1 x 0,27 mm bis 1 x 0,60 mm, in Kombination mit VPH Bohrungen von 0,00 mm bis 0,60 mm und RTP Durchmessern von 0,60 bis 1,60 mm)
- Coster Typ RKRA (Stembohrung von 1 x 0,27 mm bis 1 x 0,60 mm, in Kombination mit VPH Bohrungen von 0,00 mm bis 0,60 mm und RTP Durchmessern von 0,30 bis 1,60 mm)
- Coster Typ T (Stembohrung von 1 x 0,30 mm bis 1 x 0,70 mm)
- Coster Typ TRA (Stembohrungen von 1 x 0,30 mm bis 1 x 0,70 mm, in Kombination mit VPH Bohrungen von 0,00 mm bis 0,60 mm und RTP Durchmessern von 0,30 bis 1,60 mm)
- Coster Typ RTRA (Stembohrungen von 1 x 0,30 mm bis 1 x 0,70 mm, in Kombination mit VPH Bohrungen von 0,00 mm bis 0,60 mm und RTP Durchmessern von 0,30 bis 1,60 mm)
- Coster Typ KEN (Stembohrung von 1 x 0,27 bis 1 x 0,60 mm)
- Coster Typ RKEN (Stembohrung von 1 x 0,27 mm bis 1 x 0,60 mm, in Kombination mit VPH Bohrungen von 0,00 mm bis 0,60 mm und RTP Durchmessern von 0,30 bis 1,60 mm)
- Precision Standard (mit Stembohrungen 0.010" bis 0.024" in Kombination mit Bodenöffnungen von 0.018" bis 0.040")
- Precision Kippventil (mit Stembohrungen 0.010" bis 0.024" in Kombination mit Bodenöffnungen von 0.018" bis 0.040")
- SeaquistPerfect Ariane VX oder Ariane XT (Stembohrung von 1 x 0,25 mm bis 1 x 0,60 mm, in Kombination mit VPH Bohrungen von 0,33 mm bis 0,80 mm und Gehäusebohrungen von 0,40 bis 1,60 mm).

Als bevorzugt zur Ausführung eines erfindungsgemäßen Aerosolsprays eignen sich erfindungsgemäß Sprühköpfe mit Wirbeldüsen, besonders bevorzugt sogenannten mechanical break up (MBU) Wirbeldüsen, wie beispielsweise die Düse V06.212 der Firma Coster, die MBU Soft-Wirbeldüse .020" der Firma Precision oder der Sprühkopf WAX mit DU 25 und 27.

Je nach Sprühventil sind die Sprühraten in der Ausführungsform als Aerosolspray, bezogen auf voll gefüllte Behälter, von 1,0 bis 20,0 g/10 s möglich, bevorzugt jedoch von 6,5 bis 10,0 g/10 s.

Die Sprührate wird dabei so bestimmt, dass ein mit Treibgas und dem entsprechenden Stylingmittel gefüllter und mit dem betreffenden Ventil verschlossener Aerosolbehälter bei Raumtemperatur (etwa 23 °C) zunächst gewogen wird. Der Behälter wird samt Inhalt zehnmal kräftig von Hand geschüttelt, damit sich der Inhalt gut vermischt. Dann wird für 10 s das Ventil des senkrecht stehenden Behälters betätigt. Danach wird wiederum gewogen. Der Vorgang wird fünfmal hintereinander durchgeführt und das statistische Mittel aus den Ergebnissen gebildet. Die Differenz der beiden Wägungen ist die Sprührate pro 10 s. Daraus lässt sich durch einfaches Dividieren auch die Sprührate je Sekunde bestimmen.

Eine ganz besonders bevorzugte erfindungsgemäße Aerosolzusammensetzung ist dadurch gekennzeichnet, dass sie in einem Aerosolbehälter mit einer Versprühvorrichtung konfektioniert vorliegt.

Erfindungsgemäß wird die Aerosolzusammensetzung hergestellt, indem die filmbildenden Polymere mit einem vom Treibmittel(gemisch) unterschiedlichen organischen Lösungsmittel vermischt werden. Solche Lösungsmittel umfassen gewöhnlich ein Gemisch von Alkohole. In einigen Fällen kann auch ein nur aus ein Alkohol bestehendes System verwendet werden. Wasser ist gewöhnlich nicht vorhanden, kann aber in manchen Zubereitungen vorliegen, vorzugsweise in kleiner Menge.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Als zusätzliche Co-Solventien können organische Lösungsmittel oder ein Gemisch aus Lösungsmitteln enthalten sein. Besonders geeignet als zusätzliche Co-Solventien sind Benzylalkohol, Phenoxyethanol, unverzweigte oder verzweigte Kohlenwasserstoffe wie Pentan, Hexan, Isopentan und cyclische Kohlenwasserstoffe wie Cyclopentan und Cyclohexan. Zusätzlich zu ihren Löslichkeitseigenschaften sind die Hauptvorteile dieser Lösungsmittel ihre Fähigkeit, rasch zu trocken, ihre minimalen Auswirkung auf die Aerosolbehälter.

Eine erfindungsgemäß bevorzugte Aerosolzusammensetzung ist dadurch gekennzeichnet, dass das kosmetische Stylingmittel, bezogen auf sein Gesamtgewicht, 20 bis 95 Gew.-%, vorzugsweise von 40 bis 90 Gew.-% und insbesondere von 65 bis 85 Gew.-% ein vom Treibmittel(gemisch) unterschiedliches organisches Lösungsmittel enthält, insbesondere Ethanol oder Isopropanol oder Mischung davon.

Eine weitere erfindungsgemäß bevorzugte Aerosolzusammensetzung ist dadurch gekennzeichnet, dass das kosmetische Stylingmittel bezogen auf sein Gesamtgewicht einen Wassergehalt von 0,5 bis 20 Gew.-%, vorzugsweise von 1,0 bis 15 Gew.-% und insbesondere von 1,0 bis 10 Gew.-% aufweist.

Bevorzugt enthält das erfindungsgemäße Stylingmittel zusätzlich mindestens eine oberflächenaktive Substanz, wobei prinzipiell sowohl anionische als auch zwitterionische, ampholytische, nichtionische und kationische oberflächenaktive Substanzen geeignet sind. Die oberflächenaktiven Substanzen umfassen im Wesentlichen zwei Gruppen, die Tenside und die Emulgatoren, welche hier gemeinsam abgehandelt werden.

Eine erfindungsgemäß bevorzugte Aerosolzusammensetzung ist dadurch gekennzeichnet, dass das kosmetische Stylingmittel, bezogen auf sein Gesamtgewicht, 0,01 bis 5,0 Gew.-%, vorzugsweise 0,02 bis 4,0 Gew.-% und insbesondere 0,05 bis 2,0 Gew.-% Tenside und/oder Emulgatoren enthält.

Als anionische Tenside und Emulgatoren eignen sich in erfindungsgemäßen Mittel alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind,
- lineare und verzweigte Fettsäuren mit 8 bis 30 Kohlenstoffatomen (Seifen),
- Ethercarbonsäuren der Formel (T-I)

   R¹O-(CH₂CH₂O)_{X}CH₂-COOH (T-I)

   in der R¹ eine lineare Alkylgruppe mit 8 bis 30 Kohlenstoffatomen und x = 0 oder 1 bis 16 ist,
- Acylsarcoside mit 8 bis 24 Kohlenstoffatomen in der Acylgruppe,
- Acyltauride mit 8 bis 24 Kohlenstoffatomen in der Acylgruppe,
- Acylisethionate mit 8 bis 24 Kohlenstoffatomen in der Acylgruppe,
- Sulfobernsteinsäuremono- und -dialkylester mit 8 bis 24 Kohlenstoffatomen in der Alkylgruppe und Sulfobernsteinsäuremonoalkylpolyoxyethylester mit 8 bis Kohlenstoffatomen in der Alkylgruppe und 1 bis 6, bevorzugt 1 bis 4 Oxyethylgruppen,
- lineare Alkansulfonate mit 8 bis 24 Kohlenstoffatomen,
- lineare Alpha-Olefinsulfonate mit 8 bis 24 Kohlenstoffatomen,
- Alpha-Sulfonfettsäuremethylester von Fettsäuren mit 8 bis 30 Kohlenstoffatomen,
- Alkylsulfate und Alkylpolyglykolethersulfate der Formel (T-II)

   R²-O(CH₂-CH₂O)ₓ-SO₃H (T-II)

   in der R² eine bevorzugt lineare Alkylgruppe mit 8 bis 30 Kohlenstoffatomen und x = 0 oder 1 bis 14 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 Kohlenstoffatomen und 1 bis 6 Doppelbindungen,
- Ester der Weinsäure und Zitronensäure mit Alkoholen, welche Anlagerungsprodukte von etwa 2 bis 15 Molekülen Ethylenoxid und/oder Propylenoxid an Fettalkohole mit 8 bis 22 Kohlenstoffatomen darstellen,
- Alkyl- und/oder Alkenyletherphosphate der Formel (T-III), 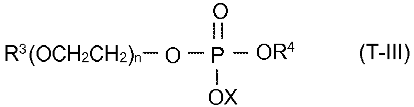 in der R³ bevorzugt ein aliphatischer Kohlenwasserstoffrest mit 8 bis 30 Kohlenstoffatomen, X Wasserstoff, ein Alkali- oder Erdalkalimetall oder NR⁵R⁶R⁷R⁸, mit R⁵ bis R⁸ unabhängig voneinander stehend für einen C₁- bis C₄-Kohlenwasserstoffrest, R⁴ Wasserstoff, ein Rest R³(CH₂CH₂O)ₙ oder X und n = 1 bis 10 ist,
- sulfatierte Fettsäurealkylenglykolester der Formel (T-IV)

   R⁹CO-(Alk-O)n-SO₃M (T-IV)

   in der R⁹ für einen linearen oder verzweigten, aliphatischen, gesättigten und/oder ungesättigten Acylrest mit 6 bis 22 Kohlenstoffatomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglycerid(ether)sulfate der Formel (T-V), 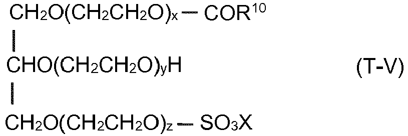 in der R¹⁰ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate in Form ihrer Natriumsalze sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure.
Vorzugsweise werden Monoglyceridsulfate der Formel (T-V) eingesetzt, in der R¹⁰ für einen linearen Acylrest mit 8 bis 18 Kohlenstoffatomen steht.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Carbonsäureamid-Derivat sowie Alkylbetaine mit 10 bis 20 Kohlenstoffatomen in der Alkylgruppe. Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂- C₁₈ - Acylsarcosin.

Erfindungsgemäß können alle dem Fachmann bekannten und üblichen kationischen Tenside verwendet werden. Dies sind insbesondere quartäre Ammoniumverbindungen, Esterquats, Amine und/oder kationisiertes Amine, Amidoamine und/oder kationisiertes Amidoamine.
Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83 und Quaternium-87 bekannten Imidazolium-Verbindungen. Die Alkylketten der oben genannten Tenside weisen bevorzugt 10 bis 18 Kohlenstoffatome auf.
Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart®, Armocare® und Quartamin® vertrieben.
Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid® S 18 im Handel erhältliche Stearamidopropyldimethylamin dar.
Das Anion der aller zuvor beschriebenen kationischen Verbindungen ist ausgewählt aus den physiologisch verträglichen Anionen. Beispielhaft hierfür seien die Halogenidionen, Fluorid, Chlorid, Bromid, Sulfat der allgemeinen Formel RSO₃⁻, worin R die Bedeutung von gesättigtem oder ungesättigtem Alkylresten mit 1 bis 4 Kohlenstoffatomen hat, oder anionische Reste organischer Säuren wie Maleat, Fumarat, Oxalat, Tartrat, Citrat, Lactat oder Acetat, genannt.

Die nichtionischen Tenside weisen als hydrophile Gruppe z. B. eine Polyolgruppe, eine Polyalkylenglykolethergruppe oder eine Kombination aus Polyol- und Polyglykolethergruppe auf. Erfindungsgemäß geeignete nichtionische Tenside und nichtionische Emulgatoren sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 Kohlenstoffatomen, an Fettsäuren mit 8 bis 30 C-Atomen und an Alkylphenole mit 8 bis 15 C-Atomen in der Alkylgruppe,
- mit einem Methyl- oder C₂- bis C₆-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 Kohlenstoffatomen, an Fettsäuren mit 8 bis 30 Kohlenstoffatomen und an Alkylphenole mit 8 bis 15 Kohlenstoffatomen in der Alkylgruppe, wie beispielsweise die unter den Verkaufsbezeichnungen Dehydol® LS, Dehydol® LT (Cognis) erhältlichen Typen,
- C₁₂-C₃₀-Carbonsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (T-VI)

   R¹¹CO-(OCH₂CHR¹²)_{w}OR¹³ (T-VI)

   in der R¹¹ für einen linearen oder verzweigten, gesättigten und/oder ungesättigten Acylrest mit 6 bis 22 Kohlenstoffatomen, R¹² für Wasserstoff oder Methyl, R¹³ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Fettsäure-N-alkylglucamide,
- Pegylierte Öle
- Glycosidisch substituierte Silikone.

Bevorzugt als Tenside und/oder Emulgatoren werden nichtionische oberflächenaktive Substanzen ausgewählt.

Erfindungsgemäß besonders bevorzugt sind Wasser-in-ÖI-Emulgatoren, insbesondere die unterschiedlichsten pegylierten Öle wie z.B PEG-30 hydrogenated Castor Öl, PEG-35 hydrogenated Castor Öl, PEG-40 hydrogenated Castor Öl und PEG-60 hydrogenated Castor Öl, sowie die Wasser-in-ÖI-Emulgatoren auf Silikonbasis.

Eine erfindungsgemäß besonders bevorzugte Gruppe von Wasser-in-ÖI-Emulgatoren auf Silikonbasis sind die Poly-(C2-C3)alkylenglycol-modifizierten Silikone, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 24, insbesondere 14 - 20, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen).

Besonders bevorzugte erfindungsgemäße Wasser in Öl Emulgatoren sind ausgewählt aus der Gruppe der alkoxylierten Siliconemulgatoren, vorzugsweise aus der Gruppe der ethoxylierten und propoxylierten Siliconemulgatoren. Besonders bevorzugt wird eine Substanz aus der Gruppe der PEG/PPG-n/m Dimethicone mit n+m >11, insbesondere mit n+m zwischen 16 und 46, vorzugsweise mit n+m zwischen 26 und 46 als Wasser in Öl Emulgator eingesetzt. Ganz besonders bevorzugt ist das PEG/PPG-18/18 Dimethicone, das z. B. in einer 1:9-Mischung mit Cyclomethicone unter der Bezeichnung Dow Corning 3225 C bzw. Dow Corning 5225 C im Handel erhältlich ist, das PEG/PPG-18/18 Dimethicone, das z. B. in einer 1:3-Mischung mit nicht-flüchtigem Dimethicone als Dow Corning ES 5227 DM Formulation Aid im Handel erhältlich ist, das PEG/PPG-4/12 Dimethicone, das z. B. unter der Bezeichnung Abil B 8852 erhältlich ist, sowie das Bis-PEG/PPG-14/14 Dimethicone, das z. B. in einer Mischung mit Cyclomethicone als Abil EM 97 (Evonik) im Handel erhältlich ist, das Bis-PEG/PPG-20/20 Dimethicone, das z. B. unter der Bezeichnung Abil B 8832 erhältlich ist, sowie das PEG/PPG-20/23 Dimethicone (z. B. Silsoft 430 und Silsoft 440).

Weitere erfindungsgemäß bevorzugte W/O-Emulgatoren auf Silikonbasis sind Poly-(C2-C3)alkylen-glycol-modifizierte Silikone, die mit C4-C18-Alkylgruppen hydrophob modifiziert sind, besonders bevorzugt Cetyl PEG/PPG-10/1 Dimethicone (früher: Cetyl Dimethicone Copolyol, z. B. erhältlich als Abil EM 90 oder in einer Mischung aus Polyglyceryl-4-isostearat, Cetyl PEG/PPG-10/1 Dimethicone und Hexyllaurat unter der Handelsbezeichnung Abil WE 09), weiterhin Alkyl Methicone Copolyol.

Bei der Auswahl erfindungsgemäß geeigneter nichtionischer ÖI-in-Wasser-Emulgatoren ist es besonders bevorzugt, ein Gemisch von nichtionischen ÖI-in-Wasser-Emulgatoren einzusetzen, um die Stabilität erfindungsgemäßer Stylingmittel optimal einstellen zu können.
Ein bevorzugter Gegenstand der vorliegenden Anmeldung ist eine Aerosolzusammensetzung, umfassend
a) ein kosmetisches Stylingmittel, enthaltend
   a1) Glycerin,
   a2) Carbonsäuregruppen-haltiges Polymer, wobei es sich bei dem Carbonsäuregruppen-haltigen Polymer zu 100 Gew.-% um ein Copolymer gebildet aus den Monomeren
      a2i) N-tert-Octylacrylamid
      a2ii) Acrylsäure
      a2iii) tert.-Butylaminoethylmethacrylsäure
         sowie gegebenenfalls weiteren Monomeren handelt,
   a3) mindestens einen Wasser in Öl Emulgator
   dadurch gekennzeichnet, dass das Verhältnis der Gewichtsanteile von Komponente a1) und Komponente a2) in dem Mittel zwischen 2:1 und 10:1 beträgt,
b) ein Treibmittel oder Treibmittelgemisch.
Gegebenfalls können in die erfindungsgemäße Aerosolzusammensetzung Additive eingearbeitet werden, um bestimmte Eigenschaften zu modifizieren. Beispiele dafür sind UV- Lichtschutzfilter mit einer Wirkung im Bereich des UV-A, UV-B und UV-C Lichtes als öllösliche Filter, Farbstoffe, Parfümöle, Duftstoffe und Riechstoffe.
Zusammenfassend weisen die erfindungsgemäßen Aerosolzusammensetzungen im Aerosolbehälter eine besonders hohe Korrosionsbeständigkeit auf, was einen großen Vorteil gegenüber dem Stand der Technik darstellt. Ferner weisen die erfindungsgemäß eingesetzten Stylingmittel eine ausgezeichnete Lagerstabilität und kosmetische Eigenschaften auf. Vorteilhaft ist insbesondere, dass der Glanz der Frisur, das Volumen und den Griff der Haare durch die Verwendung der erfindungsgemäßen Aerosolzusammensetzung zur temporären Verformung keratinischer Fasern weiterhin verbessert werden.
Ein weiterer Gegenstand der vorliegenden Erfindung ist die Verwendung einer erfindungsgemäßen Aerosolzusammensetzung zur temporären Verformung keratinischer Fasern, wobei das Glanzeffekt, die Volumenleistung und der Griff der Haare verbessert werden.
Bezüglich bevorzugter Ausführungsformen der erfindungsgemäßen Verwendungen gilt mutatis mutandis das zu den erfindungsgemäßen Mittel Gesagte.

Ein weiterer Gegenstand der vorliegenden Erfindung ist ein Verfahren zur Haarbehandlung, in dem eine erfindungsgemäße Aerosolzusammensetzung auf das Haar aufgebracht und dort bis zur nächsten Haarwäsche belassen wird.

Bezüglich bevorzugter Ausführungsformen der erfindungsgemäßen Verfahren gilt mutatis mutandis das zu den erfindungsgemäßen Mitteln Gesagte.

Die folgende Tabelle enthält Beispiele für erfindungsgemäße Stylingmittel (treibmittelfrei). Alle Zahlenwerte entsprechen, sofern nicht anders angegeben, der Menge des jeweiligen Wirkstoffs in Gewichtsprozent bezogen auf das Gesamtgewicht des Stylingmittels:

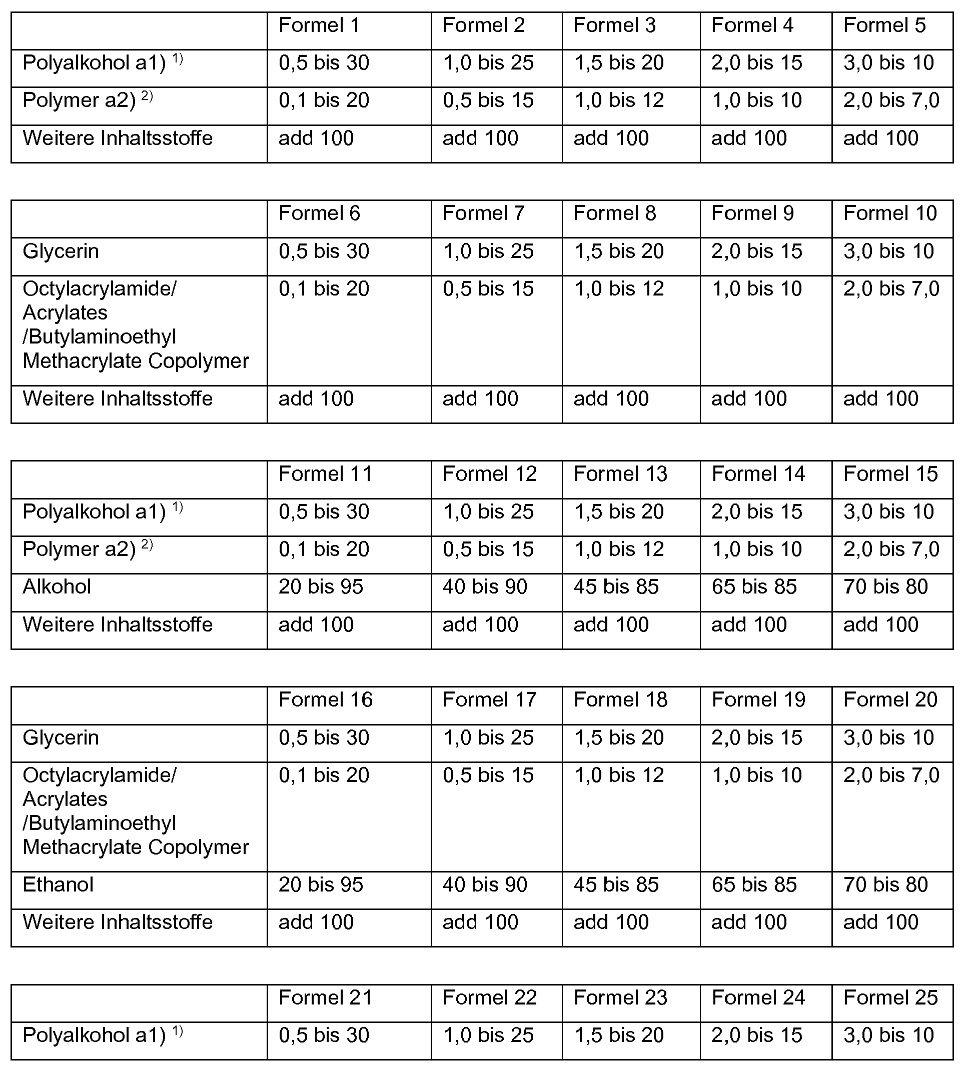

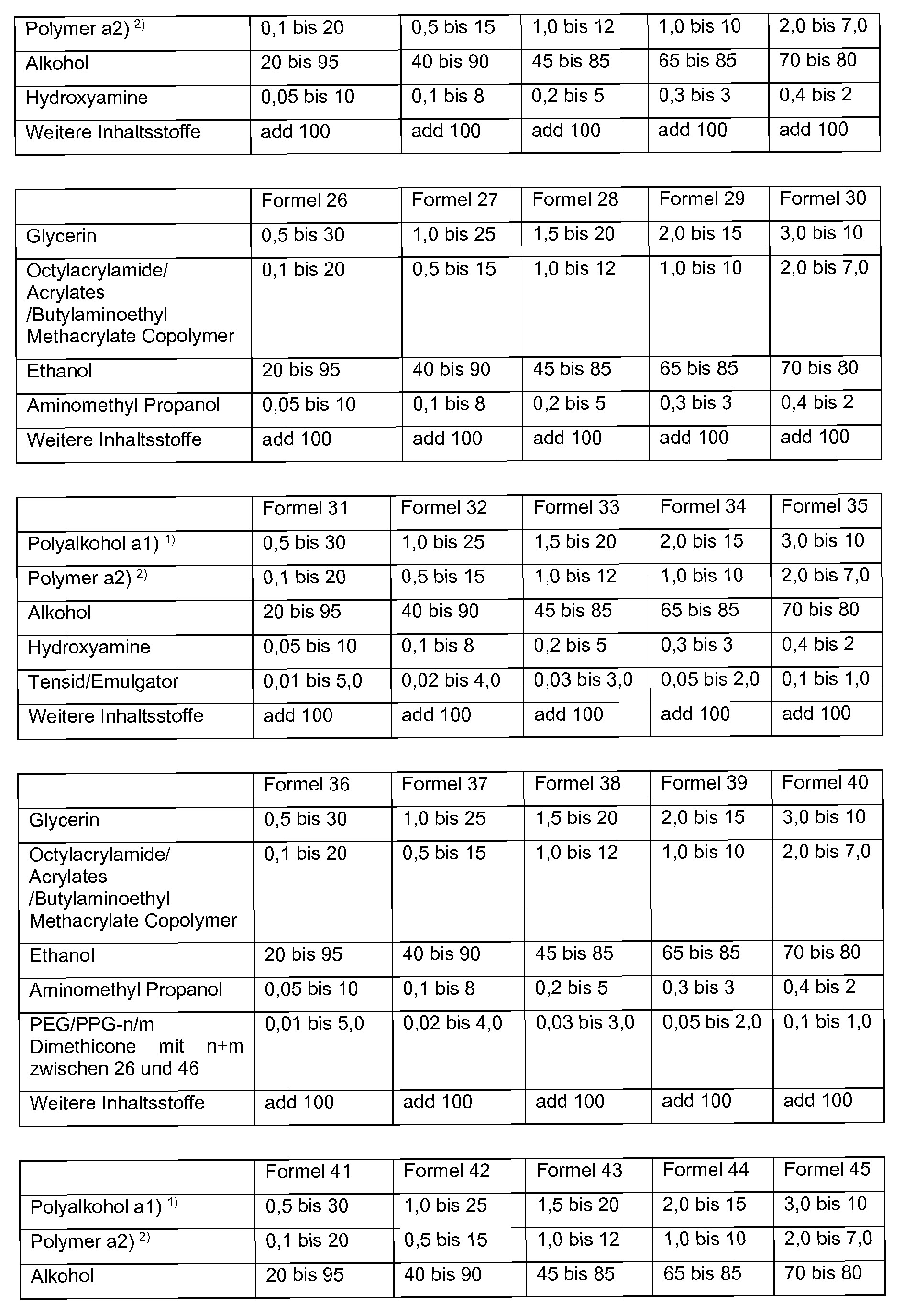

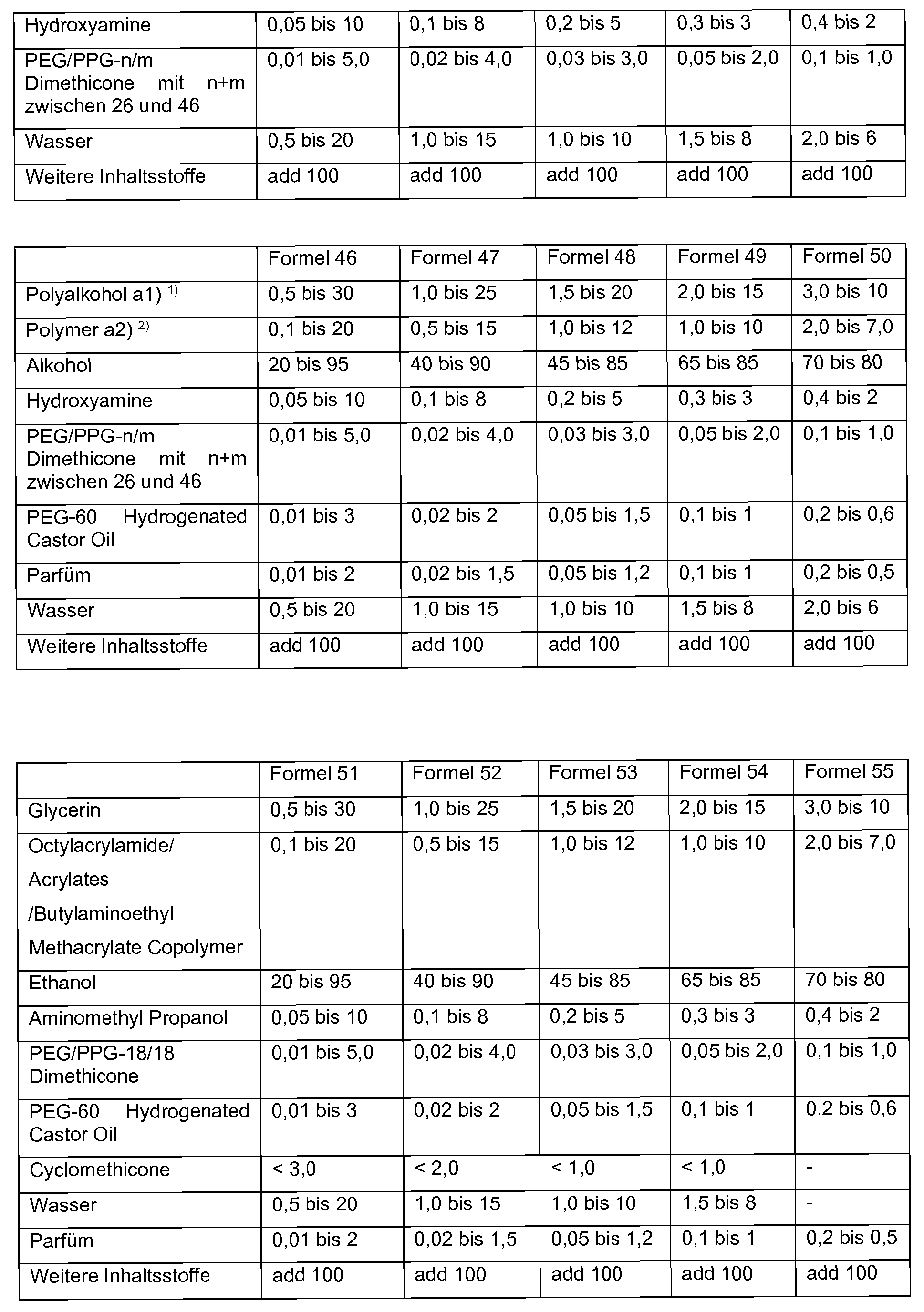

Die Aerosolprodukte lassen sich in üblicher Art und Weise herstellen. In der Regel werden alle Bestandteile des jeweiligen Stylingmittels (treibmittelfrei) in einen geeigneten druckfesten Aerosolbehälter eingefüllt. Dieser wird daraufhin mit einem Ventil verschlossen. Über herkömmliche Techniken wird schließlich die gewünschte Menge Treibmittel eingefüllt. Erfindungsgemäße Treibmittel sind ausgewählt aus den Kohlenwasserstoffen mit 3 bis 5 Kohlenstoffatomen, wie Propan, n-Butan, iso-Butan, n-Pentan und iso-Pentan, Dimethylether, Kohlendioxid, Distickstoffoxid, Fluorkohlenwasserstoffen und Fluorchlorkohlenwasserstoffen sowie Mischungen dieser Substanzen und werden in Mengen von 10 bis 85 Gew.-%, vorzugsweise 20 bis 80 Gew.-% und insbesondere 35 bis 60 Gew.-%, bezogen auf die gesamte Aerosol-zusammensetzung eingesetzt.

## Patentansprüche

1. Aerosolzusammensetzung, umfassend
a) ein kosmetisches Stylingmittel, enthaltend
a1) Glycerin,
a2) Carbonsäuregruppen-haltiges Polymer, wobei es sich bei dem Carbonsäuregruppen-haltigen Polymer zu 100 Gew.-% um ein Copolymer gebildet aus den Monomeren
a2i) N-tert-Octylacrylamid
a2ii) Acrylsäure
a2iii) tert.-Butylaminoethylmethacrylsäure
sowie gegebenenfalls weiteren Monomeren handelt,
**dadurch gekennzeichnet, dass** das Verhältnis der Gewichtsanteile von Komponente a1) und Komponente a2) in dem kosmetischen Stylingmittel zwischen 2:1 und 10:1 beträgt,
b) ein Treibmittel oder Treibmittelgemisch.

2. Aerosolzusammensetzung nach Anspruch 1, **dadurch gekennzeichnet, dass** der Gewichtsanteil der Komponente a1) am Gesamtgewicht des kosmetischen Stylingmittels zwischen 0,5 und 30 Gew.-%, vorzugsweise zwischen 1,0 und 25 Gew.-% und insbesondere zwischen 2,0 und 15 Gew.-% beträgt.

3. Aerosolzusammensetzung nach einem der vorgehenden Ansprüche, **dadurch gekennzeichnet, dass** der Gewichtsanteil der Komponente a2) am Gesamtgewicht des kosmetischen Stylingmittels zwischen 0,1 und 20 Gew.-%, vorzugsweise zwischen 0,5 und 15,0 Gew.-% und insbesondere zwischen 1,0 und 10,0 Gew.-% beträgt.

4. Aerosolzusammensetzung nach einem der vorgehenden Ansprüche, **dadurch gekennzeichnet, dass** das Treibmittel oder Treibmittelgemisch aus Dimethylether, Alkanen mit 3 bis 5 Kohlenstoffatomen und Fluorkohlenwasserstoffe ausgewählt wird.

5. Aerosolzusammensetzung nach einem der vorgehenden Ansprüche, **dadurch gekennzeichnet, dass** das Treibmittel oder Treibmittelgemisch in Mengen von 10 bis 85 Gew.-%, vorzugsweise 20 bis 80 Gew.-% und insbesondere 35 bis 60 Gew.-%, bezogen auf die gesamte Aerosol-zusammensetzung eingesetzt wird.

6. Aerosolzusammensetzung nach einem der vorgehenden Ansprüche, **dadurch gekennzeichnet, dass** das kosmetische Stylingmittel, bezogen auf sein Gesamtgewicht, 0,01 bis 5,0 Gew.-%, vorzugsweise 0,02 bis 4,0 Gew.-% und insbesondere 0,05 bis 2,0 Gew.-% Tenside und/oder Emulgatoren enthält.

7. Aerosolzusammensetzung nach einem der vorgehenden Ansprüche, **dadurch gekennzeichnet, dass** das kosmetische Stylingmittel, bezogen auf sein Gesamtgewicht, 20 bis 95 Gew.-%, vorzugsweise von 40 bis 90 Gew.-% und insbesondere von 65 bis 85 Gew.-% ein vom Treibmittel(gemisch) unterschiedliches organisches Lösungsmittel enthält.

8. Aerosolzusammensetzung nach einem der vorgehenden Ansprüche, **dadurch gekennzeichnet, dass** das kosmetische Stylingmittel bezogen auf sein Gesamtgewicht einen Wassergehalt von 0,5 bis 20 Gew.-%, vorzugsweise von 1,0 bis 15 Gew.-% und insbesondere von 1,0 bis 10 Gew.-% aufweist.

9. Verwendung einer Aerosolzusammensetzung nach einem der Ansprüche 1 bis 8 zur temporären Verformung keratinischer Fasern, wobei das Glanzeffekt, die Volumenleistung und der Griff der Haare verbessert werden.

10. Verfahren zur Haarbehandlung, in dem eine Aerosolzusammensetzung nach einem der Ansprüche 1 bis 8 auf das Haar aufgebracht und dort bis zur nächsten Haarwäsche belassen wird.

## Claims

1. An aerosol composition, comprising
a) a cosmetic styling agent, containing
a1) glycerol
a2) carboxylic acid group-containing polymer, 100 wt.% of the carboxylic acid group-containing polymer being a copolymer formed from the monomers
a2i) N-tert-octylacrylamide
a2ii) acrylic acid
a2iii) tert-butylaminoethyl methacrylic acid and possibly other monomers,
**characterized in that** the ratio of the proportions by weight of component a1) and component a2) in the cosmetic styling agent is between 2:1 and 10:1,
b) a propellant or propellant mixture.

2. The aerosol composition according to claim 1, **characterized in that** the proportion by weight of component a1) with respect to the total weight of the cosmetic styling agent is between 0.5 and 30 wt.%, preferably between 1.0 and 25 wt.%, and in particular between 2.0 and 15 wt.%.

3. The aerosol composition according to one of the preceding claims, **characterized in that** the proportion by weight of component a2) with respect to the total weight of the cosmetic styling agent is between 0.1 and 20 wt.%, preferably between 0.5 and 15.0 wt.%, and in particular between 1.0 and 10.0 wt.%.

4. The aerosol composition according to one of the preceding claims, **characterized in that** the propellant or propellant mixture is selected from dimethyl ethers, alkanes having 3 to 5 carbon atoms and hydrofluorocarbons.

5. The aerosol composition according to one of the preceding claims, **characterized in that** the propellant or propellant mixture is used in amounts of from 10 to 85 wt.%, preferably 20 to 80 wt.%, and in particular 35 to 60 wt.%, based on the total aerosol composition.

6. The aerosol composition according to one of the preceding claims, **characterized in that** the cosmetic styling agent contains, based on its total weight, from 0.01 to 5.0 wt.%, preferably 0.02 to 4.0 wt.%, and in particular 0.05 to 2.0 wt.%, of surfactants and/or emulsifiers.

7. The aerosol composition according to one of the preceding claims, **characterized in that** the cosmetic styling agent contains, based on its total weight, from 20 to 95 wt.%, preferably 40 to 90 wt.%, and in particular 65 to 85 wt.%, of an organic solvent that is different from the propellant (mixture).

8. The aerosol composition according to one of the preceding claims, **characterized in that** the cosmetic styling agent has, based on its total weight, a water content of from 0.5 to 20 wt.%, preferably 1.0 to 15 wt.%, and in particular 1.0 to 10 wt.%.

9. The use of an aerosol composition according to one of claims 1 to 8 for temporarily shaping keratin fibers, the gloss, volume performance and feel of the hair being improved.

10. A method for treating hair, wherein an aerosol composition according to one of claims 1 to 8 is applied to the hair and left there until the next hair wash.

## Revendications

1. Composition d'aérosol comprenant
a) un agent de coiffage, contenant
a1) de la glycérine,
a2) un polymère contenant des groupes d'acide carboxylique, le polymère contenant des groupes d'acide carboxylique étant à 100 % un copolymère formé à partir des monomères
a2i) n-tert-octylacrylamide
a2ii) acide acrylique
a2iii) acide tert-butylaminoéthylméthacrylique
ainsi que le cas échéant d'autres monomères,
**caractérisé en ce que** le rapport des parts de poids du composant a1) et du composant a2) dans l'agent cosmétique de coiffage est compris entre 2:1 et 10:1
b) un agent propulseur ou un mélange d'agents propulseurs.

2. Composition d'aérosol selon la revendication 1, **caractérisée en ce que** la part en poids du composant a1) dans le poids total de l'agent cosmétique de coiffage s'établit entre 0,5 et 30 % en poids, de préférence entre 1,0 et 25 % en poids et en particulier entre 2,0 et 15 % en poids.

3. Composition d'aérosol selon l'une des revendications précédentes, **caractérisée en ce que** la part en poids du composant a2) dans le poids total de l'agent cosmétique de coiffage s'établit entre 0,1 et 20 % en poids, de préférence entre 0,5 et 15,0 % en poids et en particulier entre 1,0 et 10,0 % en poids.

4. Composition d'aérosol selon l'une des revendications précédentes, **caractérisé en ce que** l'agent propulseur ou le mélange d'agents propulseurs est sélectionné parmi le diméthyléther, les alcanes comportant de 3 à 5 atomes de carbone et les fluorohydrocarbures.

5. Composition d'aérosol selon l'une des revendications précédentes, **caractérisée en ce que** l'agent propulseur ou le mélange d'agents propulseurs est utilisé dans des quantités allant de 10 à 85 % en poids, de préférence de 20 à 80 % en poids et en particulier de 35 à 60 % en poids, rapporté à l'ensemble de la composition d'aérosol.

6. Composition cosmétique selon l'une des revendications précédentes, **caractérisée en ce que** l'agent cosmétique de coiffage, rapporté à son poids total, contient de 0,01 à 5,0 % en poids, de préférence de 0,02 à 4,0 % en poids et en particulier de 0,05 à 2,0 % en poids en tensioactifs et/ou émulsifiants.

7. Composition d'aérosol selon l'une des revendications précédentes, **caractérisée en ce que** l'agent cosmétique de coiffage, rapporté à son poids total, contient de 20 à 95 % en poids, de préférence de 40 à 90 % en poids et en particulier de 65 à 85 % en poids d'un agent solvant organique différent de/du (mélange) d'agent(s) propulseur(s).

8. Composition d'aérosol selon l'une des revendications précédentes, **caractérisée en ce que** l'agent cosmétique de coiffage possède, rapporté à son poids total, une teneur en eau allant de 0,5 à 20 % en poids, de préférence de 1,0 à 15 % en poids et en particulier de 1,0 à 10 % en poids.

9. Utilisation d'une composition aérosol selon l'une des revendications 1 à 8 pour la mise en forme temporaire de fibres kératiniques, améliorant ainsi l'effet de brillance, le résultat du volume et la sensation au toucher des cheveux.

10. Procédé de traitement capillaire, dans lequel une composition d'aérosol selon l'une des revendications 1 à 8 est appliquée sur le cheveu et y est laissée à reposer jusqu'au prochain nettoyage des cheveux.
